# EUROPÄISCHE PATENTSCHRIFT

(11) **EP 0 630 637 B1**
(45) Veröffentlichungstag und Bekanntmachung des Hinweises auf die Patenterteilung: **09.12.1998**
(21) Anmeldenummer: 94107701.8
(22) Anmeldetag: 18.05.1994
(51) Int. Cl.: A61G 7/10

(54) **Transportvorrichtung für Patienten oder bettlägerige Personen**
Transporting device for patients or bedridden persons
Dispositif de transport pour patients ou personnes alitées

(30) Priorität: 14.06.1993 DE 4319524; 14.06.1993 DE 4319525
(43) Veröffentlichungstag der Anmeldung: 28.12.1994
(73) Patentinhaber: Schuster, Helmut, D-86920 Denklingen (DE)
(72) Erfinder: Schuster, Helmut, D-86920 Denklingen (DE)
(74) Vertreter: Kahler, Kurt, Dipl.-Ing.

(56) Entgegenhaltungen:
- EP-A- 0 324 237
- DE-A- 2 445 764
- DE-U- 9 015 210
- DE-U- 9 308 766
- DE-U- 9 308 767
- US-A- 3 452 371
- US-A- 3 648 305
- US-A- 4 584 989
- US-A- 5 083 625

## Beschreibung

Die Erfindung betrifft eine Transportvorrichtung für Patienten oder bettlägerige Personen. Eine Transportvorrichtung mit den Merkmalen des Oberbegriffs des Anspruches 1 ist aus US-A-5 083 625 bekannt.

Eine sich auf den ausschließlichen Transport und die Umlagerung von Patienten oder bettlägerigen Personen beziehende Vorrichtung ist aus der DE-OS 24 45 764 bekannt. Dort befindet sich an einer Längsseite eines fahrbaren Gestells ein ortsfest angebrachter Ständer mit einem Tragemechanismus. Am Tragemechanismus ist seitlich eine Liege befestigt, die mittels einer Hub- und Neigungseinrichtung vertikal auf- und abbewegbar und um eine Querachse im Bereich der Mitte der Liege schwenkbar ist. Der Tragemechanismus kann aus dem Ständer um eine parallel zur Längsrichtung der Liege verlaufenden Achse heraus- bzw. hereingeschwenkt werden. Damit wird ein Umlagern von Patienten unterstützt. Als Antrieb für den Transport ist ein Antriebsmotor vorgesehen. Bedingt durch die einseitige Belastung des fahrbaren Gestells infolge des seitlich angebrachten Ständers ist die Vorrichtung beim Transport einer Person durch verschiedene Stationen schwer verfahrbar. Außerdem weist diese Vorrichtung aufgrund der Anordnung der Räder einen großen Lenkradius und damit zur Richtungsänderung eine Anfahrphase auf, was die Verfahrbarkeit zusätzlich erschwert. Ferner behindert der Ständer bzw. Turm die Sicht bei der Fortbewegung und den seitlichen Zugang zu einem Patienten bei einer Notversorgung.

Aus dem Gebrauchsmuster DE-G 90 15 210 ist eine weitere Transportvorrichtung bekannt, bei der anstelle des seitlichen Ständers zur Aufnahme des Tragemechanismus ein ähnlicher Tragemechanismus mit vergleichbaren Funktionen wie bei der erstgenannten Vorrichtung vorgesehen ist. Der Tragemechanismus ist in einem seitlich am Gestell angeordneten, festen Turm integriert, wobei der Turm jedoch zusätzlich eine Antriebssteuereinrichtung und medizinische Geräte zur Notversorgung eines Patienten aufnimmt. Die Liege ist hierbei als abnehmbare Trage aus einem verwindungsfreien, röntgenstrahlendurchlässigen Kunststoff gefertigt. Bei einer Mitnahme der Trage, z. B. im Rettungswagen, ermöglicht die Trage einen für den Patienten umlagerungsfreien Transport, und zwar durch Umsetzen der Trage mit dem Patienten auf die Transportvorrichtung. Mit Hilfe der Transportvorrichtung kann der Patient die einzelnen Behandlungsstationen im Krankenhaus weiterhin umlagerungsfrei durchlaufen. Zwar ergeben sich bei dieser Vorrichtung Vorteile bezüglich der Notversorgung, jedoch besteht das Problem der schweren Verfahrbarkeit weiter.

Aus der EP-A-0 324 237 ist eine Vorrichtung bekannt, bei der das Steuerpult um eine vertikale Asche schwenkbar ist.

Der Erfindung liegt die Aufgabe zugrunde, eine Transportvorrichtung für Patienten oder bettlägerige Personen vorzusehen, deren Motorantriebslagerung verbessert ist, ohne daß der Zugang zum Patienten oder der bettlägerigen Person durch Steuermittel eingeschränkt ist.

Die erfindungsgemäße Transportvorrichtung für Patienten oder bettlägerige Personen ist in Anspruch 1 beschrieben.

Die erfindungsgemäße Transportvorrichtung läßt sich durch die zentrale Anordnung des Antriebsrades besser lenken. Die Anfahrphase zum Ändern der Fahrrichtung wird durch das entsprechende Verschwenken des Antriebsrades im Stillstand vermieden. Die bessere Gewichtsverteilung durch die zentrale Anordnung des Motorantriebs führt zu einer besseren Steuer- und Lenkbarkeit. Da das Steuerpult zudem bezüglich des Grundrahmens um eine vertikale Achse verschwenkbar ist, läßt sich dieses so ausrichten, daß der Zugang zum Patienten oder der bettlägerigen Person frei ist.

Vorteilhafte Ausgestaltungen der Erfindung sind Gegenstand der abhängigen Ansprüche 2 bis 12.

Wird diese Transportvorrichtung gemäß Anspruch 6 mit medizinischen Geräten ausgestattet, so ist eine ununterbrochene Notversorgung beim Transport eines Patienten sowie chirurgische Nothilfe beim Patienten möglich.

Da die Hub- und Neigungseinrichtung gemäß Anspruch 11 zwischen der Trage und dem Grundrahmen angeordnet ist, ist die Sicht beim Verfahren der Transportvorrichtung nicht eingeschränkt und der freie Zugang zum Patienten nicht behindert.

Ein Ausführungsbeispiel wird nachstehend anhand der Zeichnung näher erläutert. Es zeigen
- Fig. 1: eine Transportvorrichtung in einer perspektivischen Gesamtansicht;
- Fig. 2: einen Grundrahmen und die entsprechende Lage eines Antriebsrades in Draufsicht;
- Fig. 3: den unteren Rahmenbereich und das Antriebsrad in einer schematischen Seitenansicht;
- Fig. 4: einen Motorantrieb und einen Stellantrieb in einer Seitenansicht;
- Fig. 5: den Motorantrieb in einer Stirnansicht;
- Fig. 6: den Motorantrieb und den Stellantrieb in einer Draufsicht;
- Fig. 7: eine Richtungsanzeige- und Richtungsvorgabeeinrichtung in einer schematischen Ansicht von oben;
- Fig. 8: eine schematische Draufsicht zur Veranschaulichung der Bewegungseinrichtung der Vorrichtung und des Verschwenkens eines Steuerpultes;
- Fig. 9: den Aufbau des Steuerpultes in teilweise geschnittener Seitenansicht;
- Fig. 10: einen Querschnitt der Hub- und Neigungseinrichtung mit dem Tragrahmen und der Trage in Seitenansicht;
- Fig. 11: den Aufbau der Hub- und Neigungseinrichtung mit Tragrahmen und Trage in einer schematischen Draufsicht;
- Fig. 12: eine seitlich ausgefahrene Trage in Stirnansicht;
- Fig. 13: einen ausfahrbaren Tragrahmen in Draufsicht; und
- Fig. 14: einen Querschnitt eines Teiles des ausziehbaren Tragrahmens in Seitenansicht.

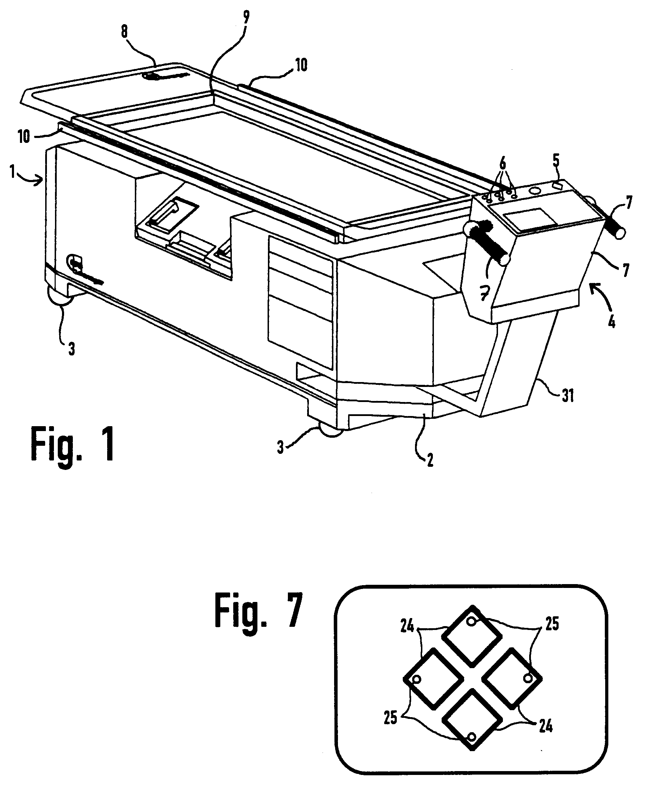

Wie aus Fig. 1 ersichtlich ist, weist die Transportvorrichtung einen Hauptkörper 1 mit einem Grundrahmen 2 auf. An den Ecken des Grundrahmens 2 befinden sich Stützräder, die als Lenkrollen 3 ausgebildet sind. An den Grundrahmen 2 ist ferner ein Steuerpult 4 angekoppelt, das verschiedene Bedienungselemente aufweist. Die Transportvorrichtung weist über dem Hauptkörper 1 einen im wesentlichen rechteckigen Tragrahmen 9 auf, der eine Trage 8 aufnehmen kann. Die Trage 8 ist für die Verwendung auf dem medizinischen Sektor zweckmäßigerweise aus einem röntgenstrahlendurchlässigen Material, beispielsweise Kunststoff, gefertigt. Die Trage 8 kann mittels einer im Hauptkörper 1 befindlichen Hub- und Neigungseinrichtung angehoben bzw. geneigt werden. Nachfolgend wird als Längsrichtung der Transportvorrichtung die Längsrichtung der Trage angesehen, als Stirnseite diejenige Seite, an der sich das Steuerpult 4 befindet.

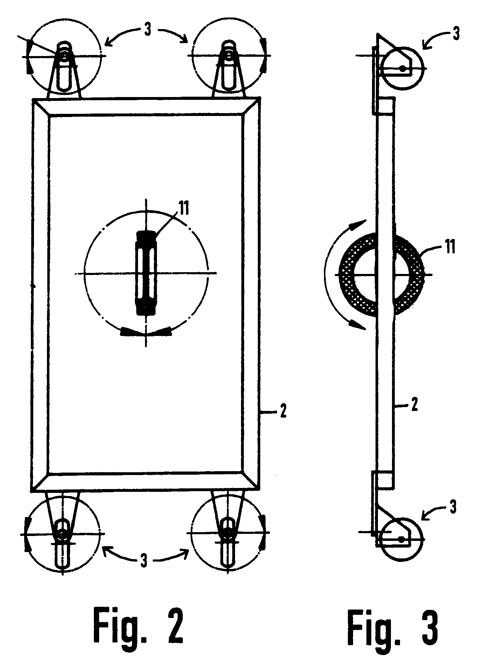

Wie aus den Fig. 2 und 3 ersichtlich ist, sind an den Ecken des Grundrahmens 2 exzentrisch gelagerte Lenkrollen 3 angebracht, während ein Antriebsrad 11 in einer mittig im Grundrahmen 2 angeordneten Aussparung aufgenommen ist. Die Lenkrollen 3 bzw. das Antriebsrad 11 sind jeweils um deren vertikale Achsen verschwenkbar gelagert, um die Änderung der Fortbewegungsrichtung zu allen Seiten direkt ohne eine Anfahrphase zu ermöglichen.

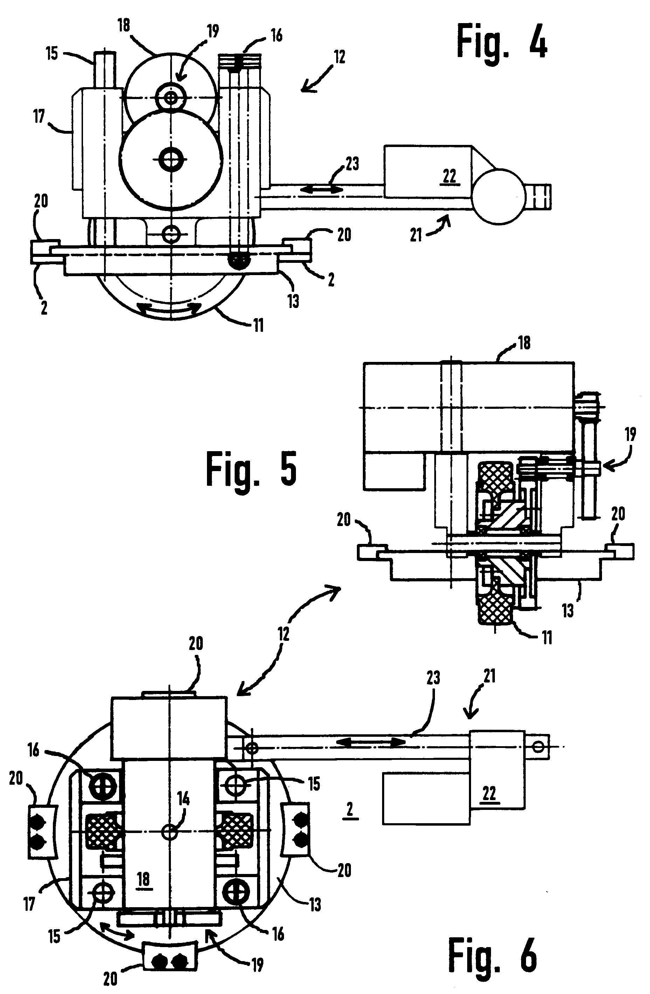

Der Motorantrieb 12 ist in den Fig. 4 bis 6 dargestellt. In die im Grundrahmen 2 vorgesehene Aussparung, insbesondere in Form einer kreisförmigen Öffnung, ist ein kreisförmig ausgebildeter Drehflansch 13 eingesetzt, der um seinen Mittelpunkt 14 drehbar ist. Der Drehflansch 13 weist eine Aussparung zur Aufnahme des Antriebsrades 11 und vertikal nach oben verlaufende Führungssäulen 15 und Federn 16 auf. Auf die Führungssäulen ist ein Motorblock 17 mit einem darin befindlichen Motor 18 vertikal beweglich aufgesetzt. Der in zwei Richtungen antreibbare Motor 18 treibt über ein Getriebe 19 das Antriebsrad 11 an. Das Getriebe 19 und das Antriebsrad 11 sind so an dem Motorblock 17 gelagert, daß diese mit dem Motorblock auf- und abbewegbar sind. Die Federn 16 sind an dem Drehflansch 13 und dem Motorblock 17 befestigt und spannen diese gegeneinander vor. Im einzelnen wird durch die nach oben gerichtete Zugkraft der Federn 16 der Drehflansch 13 gegen an dem Grundrahmen befestigte Führungselemente bzw. Führungssteine 20 gedrückt, die über den Drehflansch 13 ragen. Gleichzeitig wird der Motorblock 17 mit dem Antriebsrad 11 mittels der Federn 16 gegen den Boden gedrückt, über den die Transportvorrichtung gefahren wird, wodurch Bodenunebenheiten beim Fahrbetrieb ausgeglichen werden. Als Material zur Herstellung bietet sich für den Grundrahmen 2 z. B. Stahl an, um eine ausreichende Stabilität zu gewährleisten, und für den Drehflansch 13 z. B. Aluminium, das Gewichtsvorteile bietet und bei Serienproduktion leicht bearbeitet werden kann. Zudem wird eine Reibung von Metall auf Metall zwischen dem Drehflansch 13 und dem Grundrahmen 2 vermieden, da der Drehflansch 13 infolge der durch die Federn 16 ausgeübten Kraft an den Führungssteinen 20 reibt, die aus einem die Reibung vermindernden Material, z. B. Kunststoff, gefertigt sind.

Der Drehflansch 13 ist außerdem über einen Stellantrieb 21 mit dem Grundrahmen 2 gekoppelt. Der Stellantrieb 21 weist einen Stellantriebmotor 22 und eine Antriebstange bzw. Stellantriebspindel 23 auf. Alternativ kann der Stellantrieb 21 auch hydraulisch betrieben werden. Die Enden des Stellantriebes 21 sind jeweils drehbar am Drehflansch 13 bzw. am Grundrahmen 2 gelagert, so daß die Stellantriebspindel 23 bezüglich dem am Grundrahmen 2 vorgesehenem Lagerpunkt beim Aus- oder Einfahren der Stellantriebspindel 23 geschwenkt werden kann, um eine Drehbewegung des Drehflansches 13 um den Mittelpunkt 14 zu ermöglichen. Mit dem Drehflansch 13 dreht sich der Motorblock und damit auch das Antriebsrad um den Mittelpunkt 14. In der ausgefahrenen Stellung der Stellantriebspindel 23 steht das Antriebsrad 14 in einer Richtung, die im Vergleich zur eingefahrenen Stellung der Stellantriebspindel 23 um 90 Grad versetzt ist. Die Ausrichtung ist so zu wählen, daß in einer Stellung ein Vor- oder Rückwärtsfahren, in der anderen Stellung ein Rechts- oder Linksfahren der Transportvorrichtung möglich ist. Zur Sicherung der Ausrichtung des Antriebsrades kann eine elektromechanische oder mechanische Arretierung vorgesehen werden.

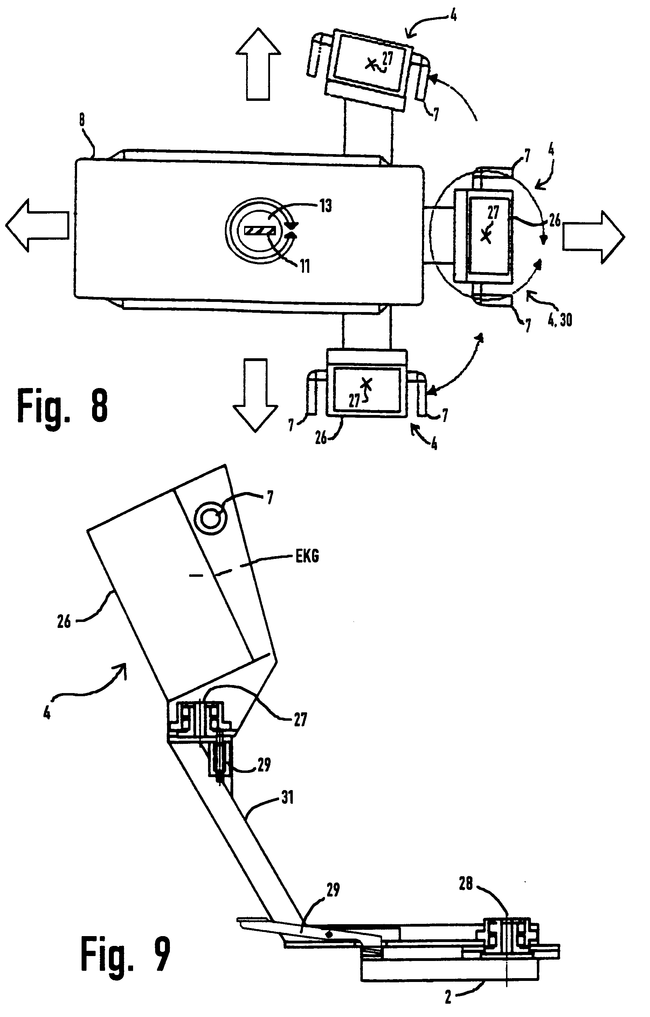

Die Fig. 7 verdeutlicht eine Richtungsanzeige- und Richtungsvorgabeeinrichtung 5, die am Steuerpult 4 befestigt ist und vier Taster 24 aufweist. Durch Drücken eines der vier Taster kann die gewünschte Fahrtrichtung der Transportvorrichtung vorgewählt werden. Zur Rückmeldung der Stellung des Antriebsrades befindet sich in jedem der Taster 24 eine Leuchtdiode 25. Der obere und der untere Taster sind für eine Fortbewegung in Vorwärts- bzw. Gegenrichtung, die beiden anderen Taster für die Fortbewegung nach rechts bzw. links vorgesehen. Beim Betätigen eines der Taster wird die Ausrichtung des Antriebsrades 11 sowie die Drehrichtung des Motors 18 festgelegt. Am Steuerpult 4 sind zwei Handgriffe 7 zum Lenken angebracht. Die Geschwindigkeit des Motors 8 kann durch Drehen eines der Handgriffe 7 (Fig. 1) über ein darin befindliches Potentiometer stufenlos eingestellt werden. Eine Richtungsänderung soll nur beim Stillstand der Transportvorrichtung möglich sein. In Fig. 1 dargestellte zusätzliche Bedienungselemente, wie die Schalter 6, dienen z. B. der Betätigung der Hub- und Kippeinrichtung für die Bewegung der Trage.

Die Bewegungsmöglichkeiten der Transportvorrichtung sind in Fig. 8 dargestellt. Durch entsprechende Auswahl der Stellung und der Drehrichtung des Antriebsrades 11 können vier Grundrichtungen festgelegt werden, wobei ein Lenken der Transportvorrichtung über Handgriffe 7 erfolgt. Das über einen Tragarm 31 am Grundrahmen 2 angekoppelte Steuerpult 4 kann bezüglich des Grundrahmens 2 um eine vertikale Achse geschwenkt werden, um insbesondere im Bereich des Kopfes des Patienten eine von jeder Seite aus freie Zugänglichkeit zu ermöglichen (siehe Fig. 8). Ferner kann im Steuerpult 4 eine Aufnahme für medizinische Geräte, beispielsweise ein EKG, vorgesehen sein. Um eine gute Ablesbarkeit der medizinischen Geräte in der Aufnahme des Steuerpultes 4 zu ermöglichen, kann auch das Steuerpult 4 zur Ausrichtung auf die Bedienungsperson am Tragarm 31 schwenkbar gelagert sein.

In Fig. 9 sind das Steuerpult 4 und der Tragarm 31 mit deren Lagerstellen 27 bzw. 28 dargestellt. Da ein Schwenken beim Fahrbetrieb nicht sinnvoll ist, ist eine Arretierungseinrichtung 29 vorgesehen, mit der das Steuerpult in einer Mittelstellung 30 an der Stirnseite der Transportvorrichtung arretiert werden kann.

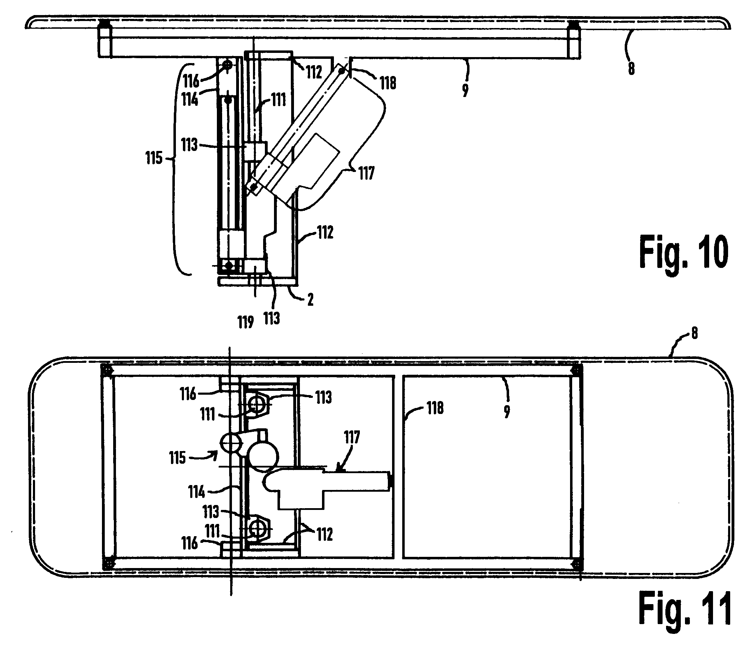

Wie aus Fig. 10 bzw. 11 ersichtlich ist, sind an dem Grundrahmen 2 vertikal verlaufende Führungssäulen 111 mit deren Fußenden befestigt. Die oberen Enden der Führungssäulen 111 sind mittels eines gleichfalls am Grundrahmen 2 befestigten Führungssäulenrahmens 112 fixiert. An den Führungssäulen 111 ist mittels Führungsbuchsen 113 ein Schlitten 114 vertikal beweglich gelagert, der sich quer zwischen dem Grundrahmen 2 und dem Tragrahmen 9 erstreckt. Ein Stellantrieb 115 ist am Grundrahmen 2 und am Schlitten 114 befestigt, so daß der Schlitten mittels des Stellantriebes 115 vertikal in eine beliebige Stellung angehoben bzw. abgesenkt werden kann. Der Schlitten 114 ist an der Oberseite an zwei Drehpunkten 116 mit dem Tragrahmen 9 gekoppelt, so daß der Tragrahmen 9 in der Längsrichtung geneigt werden kann. Am Schlitten 114 ist ein zweiter Stellantrieb 117 mit einem Ende drehbar gelagert, während das andere Ende des Stellantriebes 117 an einer im Tragrahmen 9 befindlichen Querverstrebung 118 gelagert ist. Dabei ist der Abstand zwischen der Querverstrebung 118 und dem Schlitten 114 so gewählt, daß eine stabile Lagerung des Tragrahmens 9 über drei Drehpunkte erfolgt. Befindet sich die Stellantriebspindel 119 des Stellantriebes 117 in einer mittleren Stellung, so befindet sich der Tragrahmen 9 in einer horizontalen Stellung. Wird die Stellantriebspindel 119 aus- oder eingefahren, so schwenkt der Tragrahmen 9 um die durch die Drehpunkte 116 führende Achse. Dadurch neigt bzw. hebt sich das Ende des Tragrahmens 9 und damit das der Trage 8, an dem sich zum Beispiel der Kopf des Patienten befindet. Als Kopfende des Tragrahmens 9 ist hier das Ende anzusehen, an dem sich das Steuerpult 4 befindet bzw. in den Fig. 10 und 11 die linke Seite der Zeichnung. Durch das Schwenken der Trage 8 wird eine Schocklage (Körperhochlage) oder eine Kopfhochlage eines Patienten bewirkt. Die Steuerung des Stellantriebes 115 zum Heben bzw. Senken der Trage 8 mit Hilfe des Stellantriebes 115 sowie die Steuerung des Stellantriebes 117 zum Neigen der Trage 8 erfolgt stufenlos durch das Betätigen der Schalter 6, die sich am Steuerpult befinden. Der Antrieb der Stellantriebe 115 bzw. 117 kann alternativ hydraulisch erfolgen. Schalter zum Betätigen der Stellantriebe 115 bzw. 117 sind im Steuerpult 4 vorgesehen.

Die Lage der Drehpunkte 116 kann am Tragrahmen 9 aus dem zentralen Bereich zum Kopfende hin versetzt werden, wodurch das Kopfende der Trage 8 und damit der Kopf des Patienten beim Schwenken relativ zum Körperbereich wenig bewegt wird.

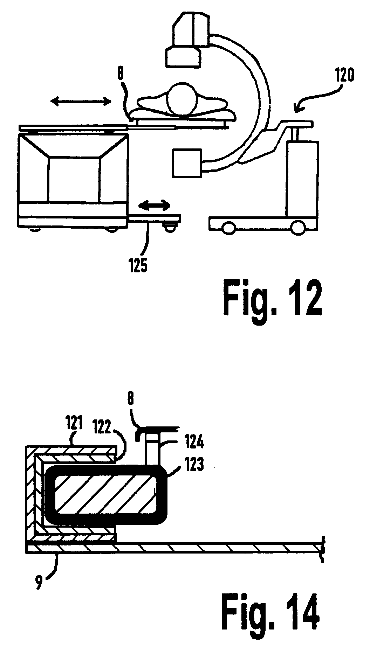

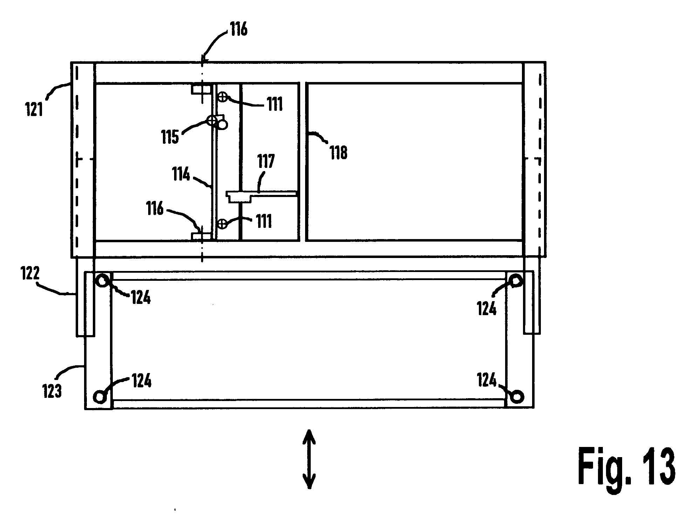

Der Tragrahmen 9 besteht aus mindestens zwei Rahmen, im vorliegenden Ausführungsbeispiel aus drei Rahmen 121 bis 123, von denen mindestens einer über Gleitelemente teleskop- bzw. schubladenartig in Querrichtung ausfahrbar ist, wie dies in den Fig. 13 bzw. 14 dargestellt ist. Auf diese Weise kann die Trage 8 mit dem Patienten ohne Umsetzen der Trage 8 direkt in z. B. ein fahrbares Röntgengerät 120 eingeschoben werden (Fig. 12). Die Trage 8 liegt in diesem Fall auf geeigneten Tragelementen 124 auf, die sich auf dem ganz ausziehbaren Rahmen 123 befinden. In den Figuren nicht dargestellte Sperr- und Rasteinrichtungen verhindern dabei ein unbeabsichtigtes Verändern der Endstellungen des Tragrahmens 9.

Um ein Kippmoment in der ausgezogenen Stellung des Tragrahmens 9 zu verhindern, kann eine Stützeinrichtung, beispielsweise eine aus dem Grundrahmen 2 ausfahrbare Stütze 125, vorgesehen werden. Um das Ausziehen des ineinander geschachtelten Rahmens 121 bis 123 mit der darauf befindlichen Trage 8 zu verhindern, ehe die Stütze 125 ausgefahren ist, kann ein weiterer Rastmechanismus vorgesehen werden, der das Ausziehen der Trage 8 erst nach dem Arretieren der ausgefahrenen Stütze 125 ermöglicht.

Zusätzliche Aufnahmen für medizinische Geräte zur Versorgung des Patienten können im Hauptkörper 1 vorgesehen werden. Außerdem können am Tragrahmen 9 Klemmschienen für die Befestigung zusätzlicher medizinischer Geräte abnehmbar befestigt werden. Für den Betrieb der gesamten Transportvorrichtung und der zusätzlichen Geräte sind Akkumulatoren vorgesehen, die mittels eines Netzanschlusses von jeder beliebigen Steckdose aus aufgeladen werden können und einen ununterbrochenen Betrieb des Antriebes und der zusätzlichen Geräte sicherstellen.

## Patentansprüche

1. Transportvorrichtung für Patienten oder bettlägerige Personen, mit
einem fahrbaren Grundrahmen (2),
einem Motorantrieb (12), der einen Motor (18) und mindestens ein im Zentrum des Grundrahmens (2) um eine vertikale Achse schwenkbar angeordnetes Antriebsrad (11) aufweist und über einen Drehflansch (13) mit dem Grundrahmen gekoppelt ist,
an den vier Ecken des Grundrahmens (2) befestigten, als Lenkrollen ausgebildeten Stützrädern (3),
einer am Grundrahmen (2) befestigten Hub- und Neigungseinrichtung,
einer mit der Hub- und Neigungseinrichtung koppelbaren Trage (8) zum Transport eines Patienten oder einer bettlägerigen Person und
einem am Grundrahmen (2) vorgesehenen Steuerpult (4) zum Steuern der Transportvorrichtung,
dadurch **gekennzeichnet,**
daß der Drehflansch (13) mindestens je eine Führungssäule (15) und Feder (16) und der Grundrahmen (2) Führungselemente (20) aufweist, wobei die Führungselemente (20) oberhalb des Drehflansches (13) am Grundrahmen (2) befestigt sind und die Feder (16) den Drehflansch (13) nach oben gegen die Führungselemente (20) und das Antriebsrad (11) nach unten zum Boden bewegt, und
daß das Steuerpult (4) bezüglich des Grundrahmens (2) um eine vertikale Achse verschwenkbar ist.

2. Transportvorrichtung nach Anspruch 1,
dadurch **gekennzeichnet,**
daß der Drehflansch (13) mit dem Grundrahmen (2) über einen Stellantrieb (21) zum Verdrehen des Drehflansches (13) und damit des Antriebsrades (11) gekoppelt ist.

3. Transportvorrichtung nach einem der Ansprüche 1 bis 2,
dadurch **gekennzeichnet,**
daß der Motor (18) in zwei Richtungen stufenlos antreibbar ist.

4. Transportvorrichtung nach einem der Ansprüche 1 bis 3,
dadurch **gekennzeichnet,**
daß das Steuerpult (4) eine Richtungsanzeige- und Richtungsvorgabeeinrichtung (5) aufweist.

5. Transportvorrichtung nach einem der Ansprüche 1 bis 4,
dadurch **gekennzeichnet,**
daß das Steuerpult (4) zum Fahrbetrieb in einer Mittelstellung an der Stirnseite des Grundrahmens (2) arretierbar ist.

6. Transportvorrichtung nach einem der Ansprüche 1 bis 5,
dadurch **gekennzeichnet,**
daß bei Anwendung als Notversorgungsmobil im Steuerpult (4) medizinische Geräte zur Versorgung des Patienten aufgenommen sind, die eine ununterbrochene Versorgung des Patienten ermöglichen.

7. Transportvorrichtung nach einem der Ansprüche 1 bis 6,
dadurch **gekennzeichnet,**
daß die Trage (8) aus röntgenstrahlendurchlässigem Kunststoff besteht.

8. Transportvorrichtung nach einem der Ansprüche 1 bis 7,
dadurch **gekennzeichnet,**
daß die Hub- und Neigungseinrichtung aus einem zum Grundrahmen (2) vertikal beweglichen, an Führungssäulen (111) geführten Schlitten (114), an dessen oberem Ende ein Rahmen (9) zum Auflegen der Trage (8) an zwei sich in Querrichtung gegenüberliegenden Lagerstellen (116) drehbar gelagert ist, einem ersten Stellantrieb (115) zum Heben bzw. Senken des Schlittens (114) bezüglich des Grundrahmens (2) und einem zweiten Stellantrieb (117), dessen eines Ende am Schlitten (114) und dessen anderes Ende mit einer weiteren Lagerstelle des Rahmens (9) drehbar gekoppelt ist,
wobei die drei Lagerstellen des Rahmens (9) ein Dreieck bilden und bei Betätigung des zweiten Stellantriebs (117) der Rahmen (9) längs einer durch die erste und zweite Lagerstelle (116) gehenden Querachse verschwenkbar ist.

9. Transportvorrichtung nach Anspruch 8,
dadurch **gekennzeichnet,**
daß die Querachse von der Mitte um einem vorbestimmten Abstand zu dem Bereich des Rahmens (9) hin verlagert ist, der bei aufgesetzter Trage (8) dem Kopfende der Trage (8) entspricht.

10. Transportvorrichtung nach Anspruch 8 oder 9,
dadurch **gekennzeichnet,**
daß die oberen Enden der Führungssäulen (111) durch einen Führungssäulenrahmen (112) abgestützt sind.

11. Transportvorrichtung nach einem der Ansprüche 8 bis 10,
dadurch **gekennzeichnet,**
daß der Rahmen (9) aus mindestens zwei Rahmen (121 bis 123) besteht, von denen wenigstens einer in Querrichtung über Gleitelemente teleskopartig ausfahrbar ist.

12. Transportvorrichtung nach Anspruch 11,
dadurch **gekennzeichnet,**
daß eine Abstützung (125) ausfahrbar ist, die einem Kippmoment der Transportvorrichtung bei ausgefahrenem Rahmen (9) entgegenwirkt.

## Claims

1. Transporting device for patients or bedridden persons, comprising
a traveling base frame (2),
a motor drive (12) including a motor (18) and at least one drive wheel (11) hinged about a vertical axis, in the center of the base frame (2), and being coupled to the base frame by a rotary flange (13),
jackwheels (3) formed as castor wheels, which are attached to the four corners of the base frame (2),
a lifting and inclining mechanism attached to the base frame (2),
a stretcher (8) coupleable to the lifting and inclining mechanism for transporting a patient or a bedridden person, and
a control panel (4) provided at the base frame (2), for controlling the transporting device,
**characterized** in that
the rotary flange (13) includes each at least one guide pillar (15) and spring (16) and the base frame (2) includes guide elements (20), said guide elements (20) being attached to the base frame (2) above the rotary flange (13) and said spring (16) moving the rotary flange (13) upwardly against the guide elements (20) and the drive wheel (11) downwardly to the ground, and
in that the control panel (4) is swiveling about a vertical axis relative to the base frame (2).

2. Transporting device according to claim 1,
**characterized** in that
the rotary flange (13) is coupled to the base frame (2) by an actuator (21) for rotating the rotary flange (13) and thus the drive wheel (11).

3. Transporting device according to any one of claims 1 to 2,
**characterized** in that
the motor (18) is driveable bidirectionally in an infinitely variable manner.

4. Transporting device according to any one of claims 1 to 3,
**characterized** in that
the control panel (4) comprises a direction indicating and direction presetting device (5).

5. Transporting device according to any one of claims 1 to 4,
**characterized** in that
the control panel (4) is lockable in a mid-position at the front side of the base frame (2) for traveling operation.

6. Transporting device according to any one of claims 1 to 5,
**characterized** in that
in case of utilization as emergency supply mobile medical instruments for supplying the patient are accommodated in the control panel (4), which allow continuously supplying the patient.

7. Transporting device according to any one of claims 1 to 6,
**characterized** in that
the stretcher (8) consists of X-ray permeable plastic.

8. Transporting device according to any one of claims 1 to 7,
**characterized** in that
the lifting and inclining mechanism consists of a slide (114) guided on guide pillars (111), which is vertically movable relative to the base frame (2), at the upper end of which a frame (9) for seating the stretcher (8) is pivoted to two transversely opposed bearings (116), a first actuator (115) for lifting and lowering the slide (114), respectively, relative to the base frame (2), and a second actuator (117), one end of which is rotatably coupled to the slide (114) and the other end of which is rotatably coupled to another bearing of the frame (9),
wherein the three bearings of the frame (9) form a triangle, and the frame (9) is swiveling along a transverse axis extending through the first and second bearings (116), upon actuation of the second actuator (117).

9. Transporting device according to claim 8,
**characterized** in that
the transverse axis is displaced a predetermined distance from the center towards the portion of the frame (9) which corresponds to the head end of the stretcher (8) when the stretcher (8) is seated.

10. Transporting device according to claim 8 or 9,
**characterized** in that
the upper ends of the guide pillars (111) are supported by a guide pillar frame (112).

11. Transporting device according to any one of claims 8 to 10,
**characterized** in that
the frame (9) consists of at least two frames (121 to 123), at least one of which is laterally extendable by slide elements in a telescope-like manner.

12. Transporting device according to claim 11,
**characterized** in that
a support (125) is extendable, which counteracts a tilting moment of the transporting device in case of extended frame (9).

## Revendications

1. Dispositif de transport pour patients ou personnes alitées comprenant - un cadre de base mobile (2),
- une commande par moteur (12) présentant un moteur (18) et au moins une roue de commande (11) placée au centre du cadre de base (2) de manière pivotante autour d'un axe vertical, et couplée au cadre de base (2) à l'aide d'un flasque rotatif (13),
- des roues de support (3) formées comme roues de guidage et fixées aux quatre coins du cadre de base (2),
- un dispositif de levage et d'inclinaison fixé sur le cadre de base (2),
- une civière (8) pouvant être coupée avec le dispositif de levage et d'inclinaison pour le transport d'un patient ou d'une personne alitée et
- un pupitre de commande (4) fixé sur le cadre de base (2), pour la commande du dispositif de transport, **caractérisé en ce que** le flasque rotatif (13) présente au moins respectivement une colonne de guidage (15) et un ressort (16), et en ce que le cadre de base (2) comprend des éléments de guidage (20), lesquels sont fixés sur le cadre de base (2) au-dessus du flasque rotatif (13), et en ce que le ressort (16) déplace le flasque rotatif (13) vers le haut contre les éléments de guidage (20), et déplace la roue de commande (11) vers le bas en direction du sol, et en ce que le pupitre de commande (4) est pivotant autour d'un axe vertical par rapport au cadre de base (2).

2. Dispositif de transport selon la revendication 1, **caractérisé en ce que** le flasque rotatif (13) est couplé au cadre de base (2) au moyen d'un servomoteur (21) pour permettre la rotation du flasque rotatif (13) et donc de la roue de commande (11).

3. Dispositif de transport selon l'une des revendications 1 à 2, **caractérisé en ce que** le moteur (18) peut être commandé en continu dans deux sens.

4. Dispositif de transport selon l'une des revendications 1 à 3, **caractérisé en ce que** le pupitre de commande (4) présente un dispositif permettant d'indiquer et de sélectionner la direction (5).

5. Dispositif de transport selon l'une des revendications 1 à 4, **caractérisé en ce que** pour la conduite, le pupitre de commande (4) peut être bloqué dans une position intermédiaire sur le front du cadre de base (2).

6. Dispositif de transport selon l'une des revendications 1 à 5, **caractérisé en ce que** lors de l'utilisation comme équipement mobile d'urgence, des appareils médicaux permettant la continuité des soins du patient sont intégrés au pupitre de commande (4).

7. Dispositif de transport selon l'une des revendications 1 à 6, **caractérisé en ce que** la civière (8) est constituée d'un matériau synthétique laissant passer les rayons X.

8. Dispositif de transport selon l'une des revendications 1 à 7, **caractérisé en ce que** le dispositif de levage et d'inclinaison est constitué d'un chariot (114) mobile verticalement et guidé sur des colonnes de guidage (111) en direction du cadre de base (2), sur l'extrémité supérieure duquel chariot un cadre (9) est fixé de manière pivotante pour poser la civière (8) sur deux points d'appui (116) opposés en sens transversal ; en ce qu'il est constitué d'un premier servomoteur (115) pour la montée ou la descente du chariot (114) par rapport au cadre de base (2) et d'un second servomoteur (117) dont l'une des extrémités est couplée de manière rotative sur le chariot (114) et l'autre extrémité à un autre point d'appui du cadre (9), les trois points d'appui du cadre (9) formant un triangle et, lorsque le second servomoteur (117) est actionné, le cadre étant pivotant le long d'un axe transversal passant par le premier et le second point d'appui (116).

9. Dispositif de transport selon la revendication 8, **caractérisé en ce que** l'axe transversal est, à partir du centre, déplacé d'une distance prédéfinie par rapport à la partie du cadre (9), distance qui, lorsque la civière (8) est installée, correspond à l'extrémité de la civière (8) qui supporte la tête.

10. Dispositif de transport selon la revendication 8 ou 9, **caractérisé en ce que** les extrémités supérieures des colonnes de guidage (111) sont soutenues par un cadre à colonnes de guidage (112).

11. Dispositif de transport selon l'une des revendications 8 à 10, **caractérisé en ce que** le cadre (9) est composé d'au moins deux cadres (121 à 123), dont un au moins peut être déployé transversalement de façon télescopique au moyen d'éléments coulissants.

12. Dispositif de transport selon la revendication 11, **caractérisé en ce qu'**un support (125) peut être déployé, évitant un moment de renversement du dispositif de transport lorsque le cadre (9) est déployé.
